(19) Europäisches Patentamt
European Patent Office
Office européen des brevets

(11) **EP 2 957 217 A1**

(12) **EUROPEAN PATENT APPLICATION**
published in accordance with Art. 153(4) EPC

(43) Date of publication:
**23.12.2015 Bulletin 2015/52**

(51) Int Cl.:
***A61B 1/04*** *(2006.01)*     ***A61B 1/00*** *(2006.01)*
***G02B 23/24*** *(2006.01)*

(21) Application number: **14810568.7**

(22) Date of filing: **10.06.2014**

(86) International application number:
**PCT/JP2014/065333**

(87) International publication number:
**WO 2014/199980 (18.12.2014 Gazette 2014/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.06.2013   JP 2013123886**

(71) Applicant: **OLYMPUS CORPORATION**
**Shibuya-ku**
**Tokyo 151-0072 (JP)**

(72) Inventors:
• **TAKAYAMA Masaki**
**Tokyo 151-0072 (JP)**

• **ABE Yuko**
**Tokyo 151-0072 (JP)**
• **TSURUOKA Takao**
**Tokyo 151-0072 (JP)**
• **TOMIOKA Makoto**
**Tokyo 151-0072 (JP)**
• **HAGIHARA Masahiro**
**Tokyo 151-0072 (JP)**

(74) Representative: **Schicker, Silvia**
**Wuesthoff & Wuesthoff**
**Patentanwälte PartG mbB**
**Schweigerstraße 2**
**81541 München (DE)**

(54) **ENDOSCOPE SYSTEM**

(57)     An endoscope system includes: an image pickup apparatus in which an insertion portion including an objective optical system is detachable, the image pickup apparatus being configured to pick up an optical image of light from an observed object transmitted by the objective optical system of the insertion portion and to output the optical image as an image pickup signal; a focus changing section provided in the image pickup apparatus and including a drive section that can change focus of the image pickup apparatus; a control section configured to generate a control signal for automatically controlling the optical image in a focused state for the drive section of the focus changing section in a state in which the insertion portion is fitted to the image pickup apparatus; and an optical characteristic acquiring section configured to acquire an optical characteristic of the insertion portion used by the control section to generate the control signal.

**FIG. 1**

**Description**

Technical Field

The present invention relates to an endoscope system, and particularly, to an endoscope system including an insertion portion detachably connected to an image pickup apparatus.

Background Art

In a medical field, surgery is conventionally conducted in which a surgical treatment is applied to an affected part in a subject, while the affected part is observed by an endoscope inserted into the subject. Further, for example, an endoscope system is conventionally used in the surgery, the endoscope system including: an insertion portion that can be inserted into a subject and that receives return light emitted from an observed object in the subject; and a camera unit in which the insertion portion is detachably connected, the camera unit picking up an image of the observed object obtained by forming an image from the return light.

More specifically, for example, Japanese Patent Application Laid-Open Publication No. 2005-334462 discloses a three-dimensional endoscope system including: a three-dimensional rigid endoscope having a function equivalent to the insertion portion described above; and a three-dimensional TV camera having a configuration including a function equivalent to the camera unit described above, wherein when an electrode group provided in the three-dimensional rigid endoscope and an electrode group provided in the three-dimensional TV camera are electrically connected, identification information used to identify a type of the three-dimensional rigid endoscope connected to the three-dimensional TV camera is acquired. Further, Japanese Patent Application Laid-Open Publication No. 2005-334462 discloses a configuration for performing focus adjustment in a picked-up image for right eye and a picked-up image for left eye based on the identification information acquired as described above. On the other hand, for example, according to an endoscope system of Japanese Patent Application Laid-Open Publication No. 2011-147707, disclosed is a configuration for performing focus adjustment according to one observation mode selected from a short-distance observation mode and a long-distance observation mode.

However, according to the three-dimensional endoscope system disclosed in Japanese Patent Application Laid-Open Publication No. 2005-334462, the identification information cannot be acquired when, for example, the electrode group is not provided in at least one of the three-dimensional rigid endoscope and the three-dimensional TV camera. As a result, the three-dimensional endoscope system disclosed in Japanese Patent Application Laid-Open Publication No. 2005-334462 has a problem that the configuration for performing the focus adjustment according to the three-dimensional rigid endoscope connected to the three-dimensional TV camera becomes complicated.

Further, while the focus adjustment based on an image pickup section substantially equivalent to a section in which the insertion portion and the camera unit are integrated is disclosed in Japanese Patent Application Laid-Open Publication No. 2011-147707, focus adjustment for an insertion portion configured to be detachably connected to the camera unit (the insertion portion and the camera unit are separately formed) is not particularly disclosed. As a result, the endoscope system disclosed in Japanese Patent Application Laid-Open Publication No. 2011-147707 has a problem that focus adjustment according to various insertion portions detachably connected to the camera unit cannot be performed.

The present invention has been made in view of the circumstances, and an object of the present invention is to provide an endoscope system that can perform, with a simple configuration, focus adjustment according to various insertion portions detachably connected to an image pickup apparatus.

Disclosure of Invention

Means for Solving the Problem

An endoscope system according to an aspect of the present invention includes: an image pickup apparatus in which an insertion portion including an objective optical system is detachable, the image pickup apparatus being configured to pick up an optical image of light from an observed object transmitted by the objective optical system of the insertion portion and to output the optical image as an image pickup signal; a focus changing section provided in the image pickup apparatus and including a drive section that can change focus of the image pickup apparatus; a control section configured to generate a control signal for automatically controlling the optical image in a focused state for the drive section of the focus changing section in a state in which the insertion portion is fitted to the image pickup apparatus; and an optical characteristic acquiring section configured to acquire an optical characteristic of the insertion portion used by the control section to generate the control signal.

Brief Description of the Drawings

Fig. 1 is a diagram showing an example of a configuration of main parts of an endoscope system according to an embodiment;
Fig. 2 is a diagram showing an example of a configuration of a camera unit in the endoscope system according to the embodiment;
Fig. 3 is a diagram showing an example of a configuration of a processor in the endoscope system according to the embodiment;
Fig. 4A is a diagram showing an example of a process executed before use of the endoscope system according to the embodiment;
Fig. 4B is a diagram showing an example of a process executed during the use of the endoscope system according to the embodiment;
Fig. 5 is a diagram showing an example of an oblique edge included in a test chart used in the process of Fig. 4A;
Fig. 6A is a diagram showing an example of the process executed before the use of the endoscope system according to the present embodiment, the example different from Fig. 4A;
Fig. 6B is a diagram showing an example of the process executed during the use of the endoscope system according to the embodiment, the example different from Fig. 4B;
Fig. 7 is a diagram for describing a configuration of an eyepiece section including a wireless tag;
Fig. 8 is a diagram for describing a configuration of a light guide post including the wireless tag;
Fig. 9 is a diagram for describing a configuration of a band member including the wireless tag; and
Fig. 10 is a diagram showing an example in which the band member of Fig. 9 is fitted to a grasping portion.

Best Mode for Carrying Out the Invention

Hereinafter, an embodiment of the present invention will be described with reference to the drawings.

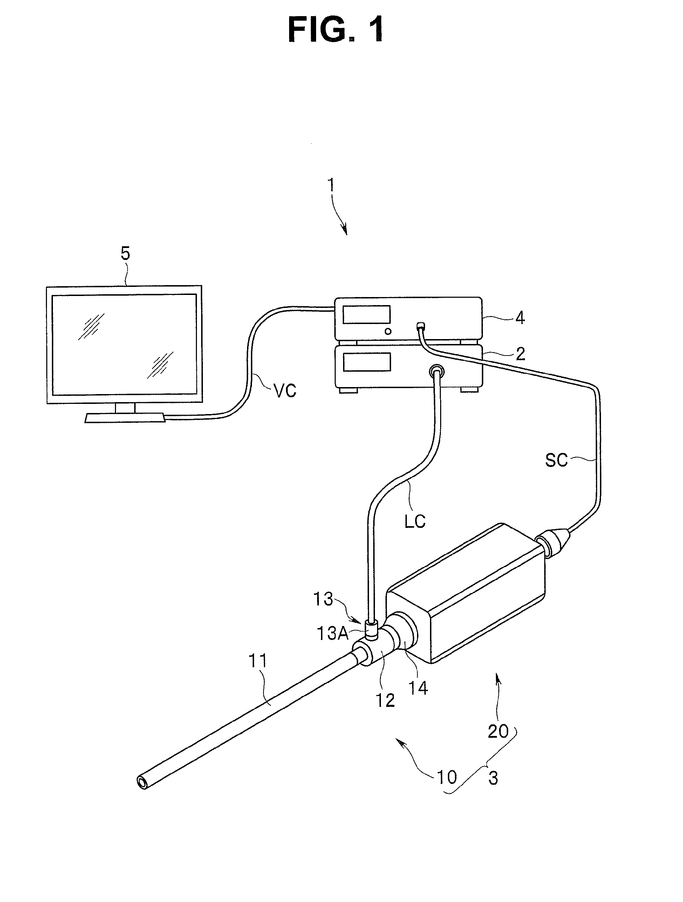

Figs. 1 to 10 are related to the embodiment of the present invention. Fig. 1 is a diagram showing an example of a configuration of main parts of an endoscope system according to the embodiment of the present invention.
As shown in Fig. 1, an endoscope system 1 includes a light source apparatus 2, a rigid endoscope image pickup apparatus 3, a processor 4, and a monitor 5.
As shown in Fig. 1, the light source apparatus 2 is configured to be able to connect to the rigid endoscope image pickup apparatus 3 through an optical cable LC. The light source apparatus 2 is also configured to be able to emit light with spectral characteristics different from each other. Furthermore, the light source apparatus 2 is configured to be able to supply, as illuminating light, light with a spectral characteristic according to an observation mode selected by operation of an observation mode changeover switch (not shown) to the optical cable LC. More specifically, for example, when a white color light observation mode is selected by the operation of the observation mode changeover switch, the light source apparatus 2 is configured to supply white color light to the optical cable LC as the illuminating light. Further, for example, when a special-light observation mode is selected by the operation of the observation mode changeover switch, the light source apparatus 2 is configured to supply special light, such as narrow band light, to the optical cable LC as the illuminating light.
As shown in Fig. 1, the rigid endoscope image pickup apparatus 3 includes a rigid endoscope 10 and a camera unit 20.
The rigid endoscope 10 has a function of an insertion portion, and the rigid endoscope 10 can be inserted into a body cavity of a test subject. The rigid endoscope 10 is configured to be detachably connected to the camera unit 20.
More specifically, for example, as shown in Fig. 1, the rigid endoscope 10 includes: a cylindrical body portion 11 formed in an elongated cylindrical shape; a grasping portion 12 provided at a rear end portion of the cylindrical body portion 11; an optical connector section 13 having a function of a connection port of the optical cable LC; and an eyepiece section 14 detachably fitted to a rear end portion of the grasping portion 12. Further, the rigid endoscope 10 is configured to be detachably connected to the camera unit 20 in a state in which the eyepiece section 14 is fitted to the rear end portion of the grasping portion 12.
A light guide (not shown) for guiding the illuminating light supplied from the light source apparatus 2 through the optical cable LC to a distal end portion of the cylindrical body portion 11 is provided inside of the cylindrical body portion 11, the grasping portion 12, and the optical connector section 13. The distal end portion (distal end face) of the cylindrical body portion 11 is provided with: an illuminating window (not shown) for applying the illuminating light transferred by the light guide to an observed object; and an objective lens (not shown) having a function of a light incident portion for receiving return light emitted from the observed object along with the application of the illuminating light. A relay optical system (not shown) having a function of an optical transfer section for transferring the return light incident on the objective lens to the rear end portion of the grasping portion 12 and including a plurality of lenses is provided inside of the cylindrical body portion 11 and the grasping portion 12.

The optical connector section 13 is configured to include a light guide post 13A detachably fitted to a side part of the grasping portion 12.

An image forming lens (not shown) having a function of an image forming section that forms an image from the return light emitted from the rear end portion of the grasping portion 12 through the relay optical system is provided inside of the eyepiece section 14.

That is, the rigid endoscope image pickup apparatus 3 of the present embodiment is configured so that the return light formed by the rigid endoscope 10 enters the camera unit 20 connected to the rear end portion of the rigid endoscope 10.

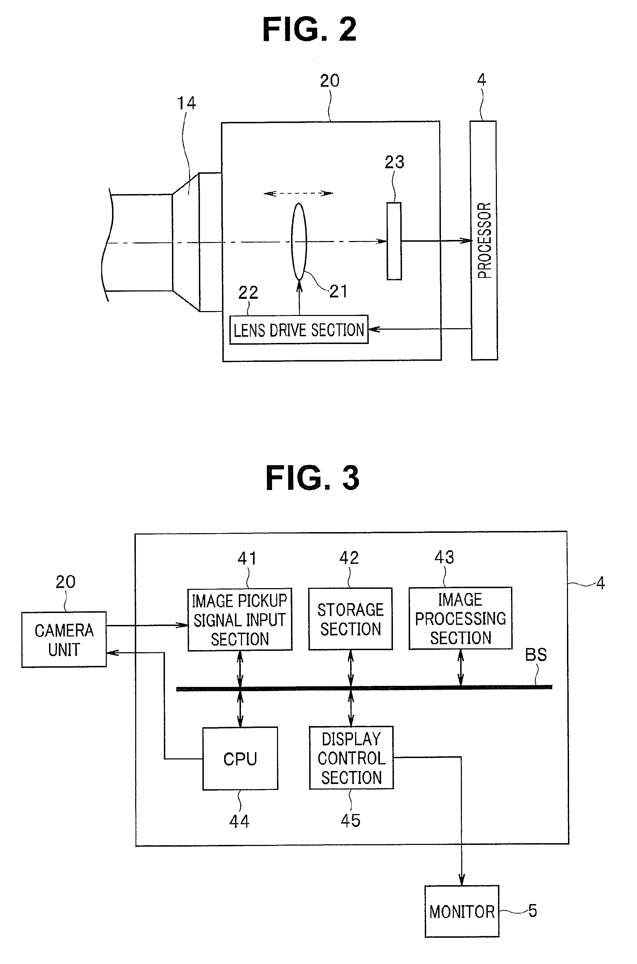

As shown in Fig. 1, the camera unit 20 is configured to be able to connect to the processor 4 through a signal cable SC provided at a rear end portion. Further, an optical window (now shown) for receiving light from outside is provided at a part of connection with the eyepiece section 14, on a distal end face of the camera unit 20. Further, as shown in Fig. 2, the camera unit 20 is configured to include a focus lens 21, a lens drive section 22, and an image pickup device 23. Fig. 2 is a diagram showing an example of a configuration of a camera unit in the endoscope system according to the embodiment.

The focus lens 21 is configured to be able to perform focus adjustment of an optical image picked up by the image pickup device 23 by moving on an optical axis within a predetermined movable range according to drive of the lens drive section 22.

The lens drive section 22 is configured to be able to drive the focus lens 21 based on a lens drive control signal outputted from the processor 4. The lens drive section 22 is configured to be able to move the focus lens 21 in an optical axis direction by driving the focus lens 21.

According to the configurations of the focus lens 21 and the lens drive section 22 as described above, the lens drive section 22 can move (drive) the focus lens 21 in the optical axis direction based on the lens drive control signal from the processor 4 to put the optical image picked up by the image pickup device 23 into a focused state. That is, the focus lens 21 and the lens drive section 22 have a function of a focus adjustment section that performs action regarding the focus adjustment of the camera unit 20.

The image pickup device 23 is configured to receive, through an image pickup surface, an optical image according to light passing through an image pickup lens group and to pick up the received optical image to generate an image pickup signal. Then, the image pickup signal generated by the image pickup device 23 is outputted to the processor 4 (through the signal cable SC).

As shown in Fig. 1, the processor 4 is configured to be able to connect to the monitor 5 through a video cable VC. Further, as shown in Fig. 3, the processor 4 is configured to include an image pickup signal input section 41, a storage section 42, an image processing section 43, a CPU 44, and a display control section 45. Fig. 3 is a diagram showing an example of a configuration of a processor in the endoscope system according to the embodiment.

The image pickup signal input section 41 is configured to generate image data by applying signal processing, such as noise removal and A/D conversion, to the image pickup signal outputted from the camera unit 20. Then, the image data generated by the image pickup signal input section 41 is outputted to the image processing section 43 and the CPU 44 through a bus BS.

The storage section 42 is configured to include, for example, a non-volatile memory. The storage section 42 is configured to be able to store various data, such as programs and databases, used for processing by the CPU 44.

The image processing section 43 is configured to apply various image processing to the image data generated by the image pickup signal input section 41. Then, the image data after the image processing by the image processing section 43 is outputted to the storage section 42 and the display control section 45 through the bus BS.

The CPU 44 is configured to be able to cause each component of the processor 4 to perform action according to, for example, a program read from the storage section 42 and operation of an input interface (not shown) such as a switch. The CPU 44 is configured to be able to control each component of the processor 4 through the bus BS or a signal line (not shown).

On the other hand, the CPU 44 having a function of a control section is configured to be able to execute a process described later to generate a lens drive control signal for performing focus adjustment according to the rigid endoscope 10 used at the same time as the camera unit 20 and to output the generated lens drive control signal to the lens drive section 22 of the camera unit 20 (through the signal cable SC).

The display control section 45 is configured to generate a video signal by applying various processes according to control by the CPU 44 and the like to the image data after the image processing by the image processing section 43. Then, the video signal generated by the display control section 45 is outputted to the monitor 5 (through the video cable VC).

Note that, according to the present embodiment, the endoscope system 1 may be formed so that, for example, one or more of each component of the processor 4 are provided in the camera unit 20.

The monitor 5 is configured to be able to display, on a screen, an image and the like according to the video signal outputted from the processor 4 through the video cable VC.

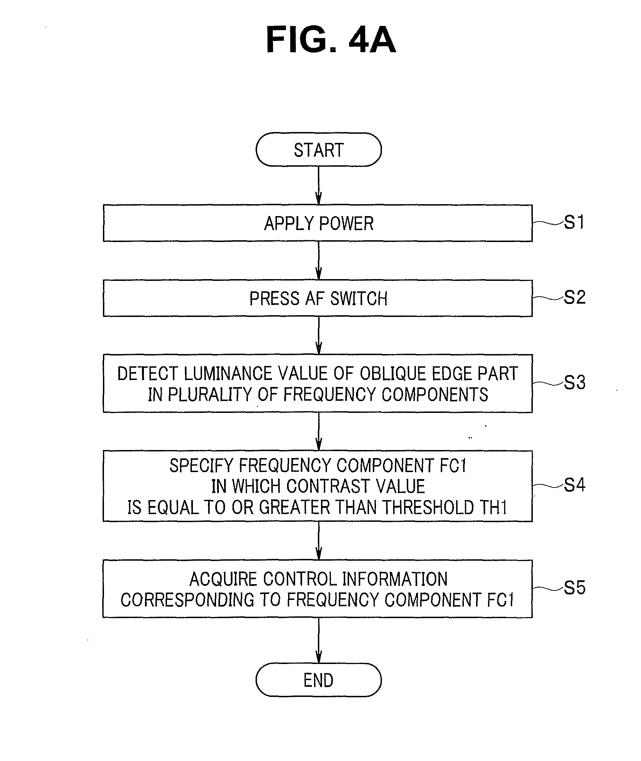

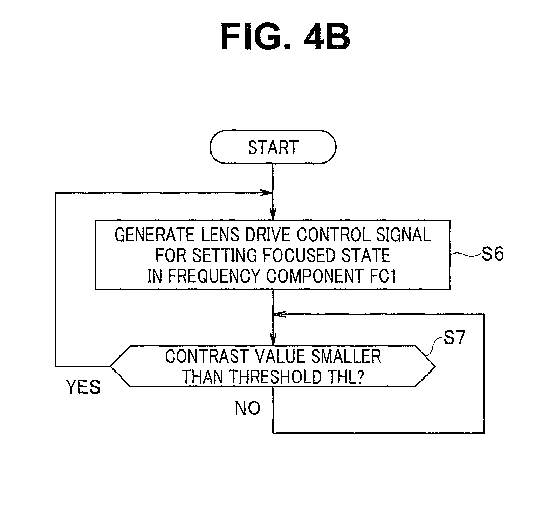

Next, an example of a case in which processes shown in Fig. 4A and Fig. 4B are executed in the endoscope system 1 of the present embodiment will be described. Fig. 4A is a diagram showing an example of a process executed before use of the endoscope system according to the embodiment. Fig. 4B is a diagram showing an example of a process executed during the use of the endoscope system according to the embodiment.

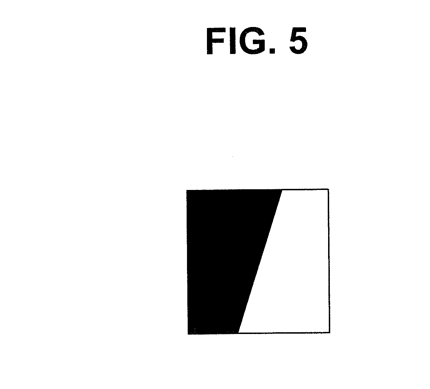

First, a user, such as a surgeon, connects each component of the endoscope system 1 as shown in Fig. 1 and applies power (step S1 of Fig. 4A). Subsequently, the user arranges the distal end portion of the cylindrical body portion 11 so that an image of a predetermined test chart including a white and black oblique edge as shown for example in Fig. 5 can be picked up and presses an AF switch (not shown) provided in the camera unit 20 (step S2 of Fig. 4A). Fig. 5 is a diagram showing an example of the oblique edge included in the test chart used in the process of Fig. 4A.

Subsequently, when the CPU 44 acquires image data in which the image of the predetermined test chart is picked up, the CPU 44 detects a luminance value of a white and black oblique edge part in each of a plurality of predetermined frequency components of the acquired image data (step S3 of Fig. 4A). More specifically, for example, the CPU 44 detects the luminance value of the white and black oblique edge part in each of three frequency components that are set in advance so that FD1<FD2<FD3.

Note that the frequency components denote spatial frequency components in the present embodiment. Here, the spatial frequency components are generally used as parameters indicating pitches of gradation change on the image. Further, one image generally includes a plurality of spatial frequency components. Therefore, for example, intervals of gradation are wide in regions with many low-frequency components in an image including the plurality of spatial frequency components, and on the other hand, intervals of gradation are narrow in an image with many highfrequency components in the image. Then, for example, the CPU 44 of the present embodiment dissolves the acquired image data into each frequency component and executes a process of detecting the luminance value of each of the dissolved frequency components to obtain a processing result of step S3 of Fig 4A.

The CPU 44 having a function of a frequency component specifying section specifies a frequency component in which a difference between the luminance value of white and the luminance value of black is equal to or greater than a predetermined threshold TH1, that is, a frequency component FC1 in which a contrast value is equal to or greater than the predetermined threshold TH1, from among the plurality of predetermined frequency components used in the process of step S3 of Fig. 4A (step S4 of Fig. 4A). Then, the CPU 44 having a function of an optical characteristic acquiring section acquires the frequency component FC1 as an optical characteristic of the rigid endoscope 10 in the process of step S4 of Fig. 4A.

More specifically, for example, when the CPU 44 detects that the difference between the luminance value of white and the luminance value of black is equal to or greater than the predetermined threshold TH1 in the frequency component FD1 among the three frequency components FD1, FD2, and FD3, the CPU 44 specifies the frequency component FD1 as the frequency component FC 1. Then, through such a process, the frequency component FD1 suitable for the rigid endoscope 10 connected to the camera unit 20, that is, the rigid endoscope 10 used in combination with the camera unit 20, can be specified. In other words, according to the present embodiment, the plurality of predetermined frequency components used in the process of step S3 of Fig. 4A need to be set separately from each other to an extent that one frequency component FC1 can be specified in the process of step S4 of Fig. 4A.

The CPU 44 having a function of a control information acquiring unit acquires control information used to generate a lens drive control signal corresponding to the frequency component FC1 specified in step S4 of Fig. 4A (step S5 of Fig. 4A). More specifically, for example, the CPU 44 acquires control information including at least a value 1/2 times (or substantially 1/2 times) the frequency component FC1 specified in step S4 of Fig. 4A, as the control information used to generate the lens drive control signal. That is, according to the process of step S5 of Fig. 4A, different control information is acquired according to the size of the frequency component FC1 specified in the process of step S4 of Fig. 4A.

On the other hand, for example, when the user recognizes completion of the series of processes shown in Fig. 4A by checking an image displayed on the monitor 5, the user performs operation for arranging the distal end portion of the cylindrical body portion 11 in the vicinity of a desired observed site in a body cavity of a test subject.

Based on the control information acquired in step S5 of Fig. 4A, the CPU 44 generates and outputs a lens drive control signal for putting the optical image picked up by the camera unit 20 (image pickup device 23) into the focused state in the frequency component FC1 (step S6 of Fig. 4B).

Based on the image data inputted after the execution of the process of step S6 of Fig. 4B, the CPU 44 judges whether the contrast value of the image data is smaller than the predetermined threshold THL (<TH1) (step S7 of Fig. 4B). Then, according to the process of step S7 of Fig. 4B, the fact that the optical image picked up by the camera unit 20 (image pickup device 23) is in the focused state is indicated when, for example, a judgement result indicating that the contrast value of the image data is equal to or greater than the predetermined threshold THL is obtained. Further, according to the process of step S7 of Fig. 4B, the fact that the optical image picked up by the camera unit 20 (image pickup device 23) is deviated from the focused state is indicated when, for example, a judgement result indicating that the contrast value of the image data is smaller than the predetermined threshold THL is obtained.

Then, when the judgement result indicating that the contrast value of the image data is equal to or greater than the threshold THL is obtained in the process of step S7 of Fig. 4B, the CPU 44 executes the process of step S7 of Fig. 4B again based on next image data inputted after the judgement result is obtained.

That is, when the judgement result indicating that the contrast value of the image data is equal to or greater than the predetermined threshold THL is obtained in the process of step S7 of Fig. 4B, a new lens drive control signal is not generated. Therefore, an arrangement position of the focus lens 21 is maintained at a current position.

Further, when the CPU 44 obtains the judgement result indicating that the contrast value of the image data is smaller than the predetermined threshold THL in the process of step S7 of Fig. 4B, the CPU 44 returns to step S6 of Fig. 4B to execute the process.

That is, when the judgement result indicating that the contrast value of the image data is smaller than the predetermined threshold THL is obtained in the process of step S7 of Fig. 4B, the arrangement position of the focus lens 21 is moved from the current position according to the lens drive control signal newly generated in the process of step S6 of Fig. 4B.

As described, according to the processes of Fig. 4A and Fig. 4B, the lens drive control signal for putting the optical image picked up by the camera unit 20 (image pickup device 23) into the focused state is generated and outputted based on the image data obtained when an image of the predetermined test chart is picked up in the state in which the rigid endoscope 10 is connected to the camera unit 20. Therefore, according to the processes of Fig. 4A and Fig. 4B, the frequency component FC1 can be individually acquired for each of various insertion portions detachably connected to the image pickup apparatus, and focus adjustment according to the insertion portion can be performed with a simple configuration.

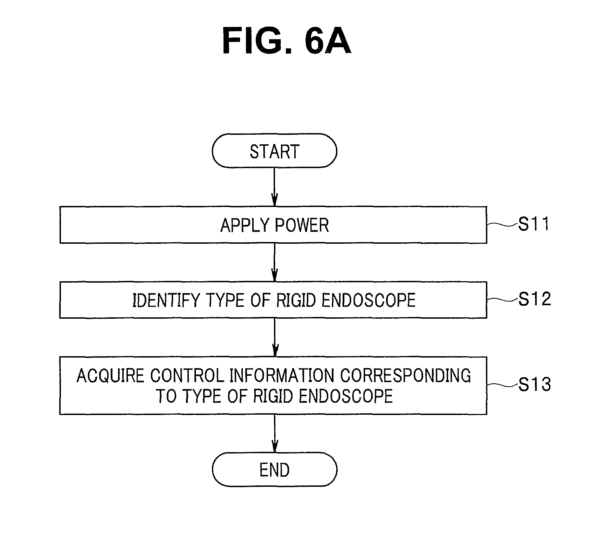

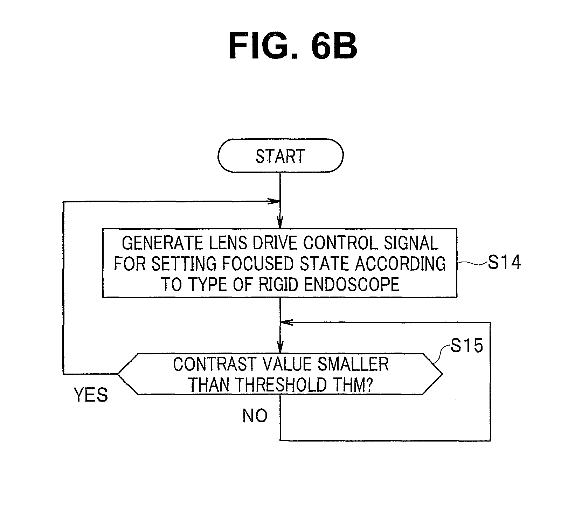

Note that, according to the present embodiment, for example, a type of the rigid endoscope 10 used at the same time as the camera unit 20 may be identified, and a lens drive control signal for performing focus adjustment may be generated based on the identified type of the rigid endoscope 10. A process executed in such a case will be described with reference mainly to flowcharts of Fig. 6A and Fig. 6B. Fig. 6A is a diagram showing an example of the process executed before the use of the endoscope system according to the embodiment, the example different from Fig. 4A. Fig. 6B is a diagram showing an example of the process executed during the use of the endoscope system according to the embodiment, the example different from Fig. 4B.

First, the user, such as a surgeon, connects each component of the endoscope system 1 as shown in Fig. 1 and applies power (step S11 of Fig. 6A).

Subsequently, before connecting the rigid endoscope 10 to the camera unit 20, the user arranges the camera unit 20 on a position that allows an image of a part (for example, the eyepiece section 14) of an outer surface of the rigid endoscope 10 displaying an identification code (one-dimensional code or two-dimensional code) including the information regarding the type of the rigid endoscope 10 to be picked up, while checking the image displayed on the monitor 5.

The CPU 44 having a function of an identification section reads the identification code included in the image data based on the image data generated by the image pickup signal input section 41 to identify the type of the rigid endoscope 10 used at the same time as the camera unit 20 (step S12 of Fig. 6A).

On the other hand, along with the execution of the process of step S12 of Fig. 6A, the CPU 44 reads, from the storage section 42, a database DB1 including a plurality of data associating the type of one rigid endoscope detachably connected to the camera unit 20 and the control information used in the focus adjustment in the one rigid endoscope. Note that the control information included in the database DB1 includes optical characteristics, such as a depth of field of one rigid endoscope detachably connected to the camera unit 20, an evaluation region in the focus adjustment of the one rigid endoscope, and a frequency component FC2 in which the contrast value of the image data obtained when the one rigid endoscope is used to pick up an image of the observed object is equal to or greater than a predetermined threshold TH2. Further, the data, such as the control information, included in the database DB1 is stored in advance in the storage section 42 at a timing before the camera unit 20 is used, such as at factory shipment. That is, the CPU 44 having the function of the optical characteristic acquiring section acquires the optical characteristics corresponding to the type of the rigid endoscope 10 identified in the process of step S12 of Fig. 6A.

The CPU 44 having the function of the control information acquiring section refers to the database DB1 read from the storage section 42 to acquire the control information corresponding to the type of the rigid endoscope 10 identified in step S12 of Fig. 6A (step S13 of Fig. 6A).

On the other hand, for example, when the user recognizes completion of the series of processes shown in Fig. 6A by checking the image displayed on the monitor 5, the user performs operation for arranging the distal end portion of the cylindrical body portion 11 in the vicinity of the desired observed site in the body cavity of the test subject.

The CPU 44 generates and outputs a lens drive control signal for setting the focused state according to the type of the rigid endoscope 10 used at the same time as the camera unit 20 based on the control information acquired in step S13 of Fig. 6A (step S14 of Fig. 6B). That is, according to the process of step S14 of Fig. 6B, the evaluation region corresponding to the type of the rigid endoscope 10 identified in step S12 of Fig. 6A is set in the image data, and the control signal for putting the set evaluation region into the focused state is generated.

The CPU 44 judges whether the contrast value of the image data is smaller than a predetermined threshold THM (<TH2) based on the image data inputted after the execution of the process of step S14 of Fig. 6B (step S15 of Fig. 6B).

Then, when a judgement result indicating that the contrast value of the image data is equal to or greater than the predetermined threshold THM is obtained in the process of step S15 of Fig. 6B, the CPU 44 executes the process of step S 15 of Fig. 6B again based on next image data inputted after the judgement result is obtained.

That is, when the judgement result indicating that the contrast value of the image data is equal to or greater than the predetermined threshold THM is obtained in the process of step S15 of Fig. 6B, a new lens drive control signal is not generated. Therefore, the arrangement position of the focus lens 21 is maintained at the current position.

Further, when a judgement result indicating that the contrast value of the image data is smaller than the predetermined threshold THM is obtained in the process of step S15 of Fig. 6B, the CPU 44 returns to step S14 of Fig. 6B and executes the process.

That is, when the judgment result indicating that the contrast value of the image data is smaller than the predetermined threshold THM is obtained in the process of step S15 of Fig. 6B, the arrangement position of the focus lens 21 is moved from the current position according to the lens drive control signal newly generated in the process of step S14 of Fig. 6B.

As described, according to the processes of Fig. 6A and 6B, the type of the rigid endoscope 10 used at the same time as the camera unit 20 is identified based on the image data obtained by picking up an image of the code displayed at the predetermined part of the rigid endoscope 10, and the lens drive control signal for setting the focused state according to the identified type of the rigid endoscope 10 is generated and outputted. Therefore, according to the processes of Fig. 6A and Fig. 6B, the frequency component FC2 can be individually acquired for each of various insertion portions detachably connected to the image pickup apparatus, and the focus adjustment according to the insertion portion can be performed with a simple configuration.

Note that, according to the present embodiment, a process described below or the like can be executed in a period from step S11 to step S12 of Fig. 6A to identify the type of the rigid endoscope 10 used at the same time as the camera unit 20.

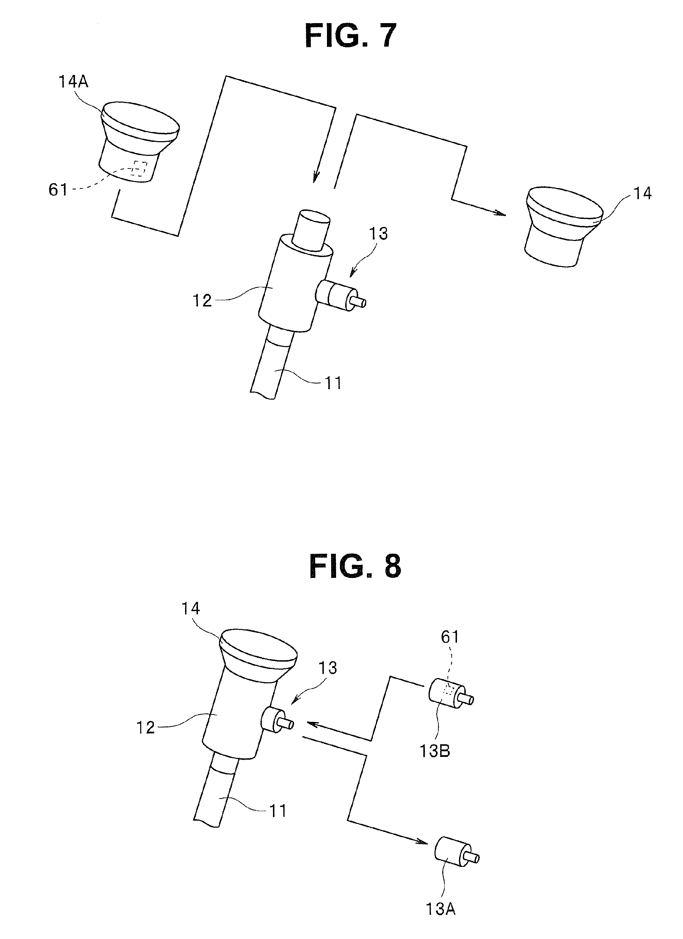

For example, the user, such as a surgeon, installs an eyepiece section 14A as shown in Fig. 7 in place of the eyepiece section 14 after connecting each component of the endoscope system 1 as shown in Fig. 1 and applying power. Fig. 7 is a diagram for describing a configuration of an eyepiece section including a wireless tag.

As shown in Fig. 7, the eyepiece section 14A has the same shape and function as the eyepiece section 14, and the eyepiece section 14A is configured to be detachably fitted to the rear end portion of the grasping portion 12 in place of the eyepiece section 14. Further, as shown in Fig. 7, the eyepiece section 14A is provided with a wireless tag 61 having a function of a wireless transmission section that can transmit, as a wireless signal, ID information associated with the type of the rigid endoscope 10.

That is, according to the configuration of the eyepiece section 14A described above, the ID information associated with the type of the rigid endoscope 10 is transmitted from the wireless tag 61 as a wireless signal. Therefore, the CPU 44 can identify the type of the rigid endoscope 10 used at the same time as the camera unit 20 based on the ID information included in the wireless signal received by, for example, a wireless reception section (not shown) of the camera unit 20.

Alternatively, for example, the user, such as a surgeon, installs a light guide post 13B as shown in Fig. 8 in place of the light guide post 13A after connecting each component of the endoscope system 1 as shown in Fig. 1 and applying power. Fig. 8 is a diagram for describing a configuration of a light guide post including a wireless tag.

As shown in Fig. 8, the light guide post 13B has the same shape and function as the light guide post 13A, and the light guide post 13B is configured to be detachably fitted to the side part of the grasping portion 12 in place of the light guide post 13A. Further, as shown in Fig. 8, the light guide post 13B is provided with the wireless tag 61 having the function of the wireless transmission section that can transmit, as a wireless signal, the ID information associated with the type of the rigid endoscope 10.

That is, according to the configuration of the light guide post 13B described above, the ID information associated with the type of the rigid endoscope 10 is transmitted from the wireless tag 61 as a wireless signal. Therefore, for example, the CPU 44 can identify the type of the rigid endoscope 10 used at the same time as the camera unit 20 based on the ID information included in the wireless signal received by the wireless reception section of the camera unit 20.

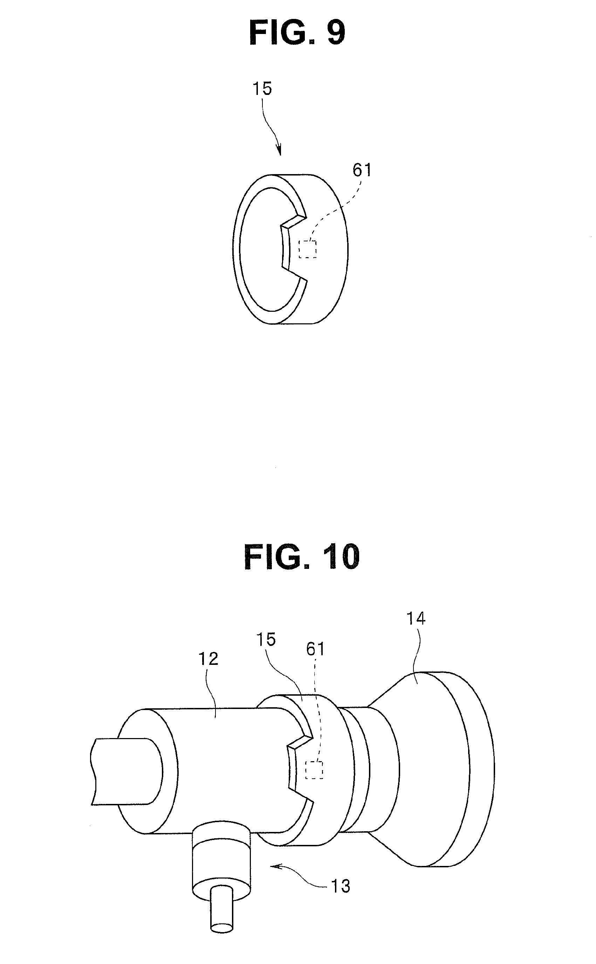

Furthermore, for example, the user, such as a surgeon, fits a band member 15 as shown in Fig. 9 to the grasping portion 12 after connecting each component of the endoscope system 1 as shown in Fig. 1 and applying power. Fig. 9 is a diagram for describing a configuration of a band member including a wireless tag.

The band member 15 is formed by, for example, an elastic member, such as rubber, and the band member 15 is configured to be detachably fitted to the grasping portion 12 in an aspect as shown in Fig. 10. Further, as shown in Fig. 9, the band member 15 is provided with the wireless tag 61 having the function of the wireless transmission section that can transmit, as a wireless signal, the ID information associated with the type of the rigid endoscope 10. Fig. 10 is a diagram showing an example of a case in which the band member of Fig. 9 is fitted to the grasping portion.

That is, according to the configuration of the band member 15, the ID information associated with the type of the rigid endoscope 10 is transmitted from the wireless tag 61 as a wireless signal. Therefore, the CPU 44 can identify the type of the rigid endoscope 10 used at the same time as the camera unit 20 based on the ID information included in the wireless signal received by, for example, the wireless reception section of the camera unit 20.

Note that, according to the present embodiment, for example, the test chart shown in Fig. 5 may be depicted on an inner circumferential face of a white balance cap used in white balance adjustment.

Further, according to the present embodiment, for example, when the illuminating light supplied from the light source apparatus 2 is switched by operation of the observation mode changeover switch after the control information is obtained in the process of step S5 of Fig. 4A or step S12 of Fig. 6A, the control information suitable for the illuminating light after the switch can be obtained without execution of the same process again. More specifically, for example, new control information suitable for the illuminating light after the switch can be obtained by executing a process of shifting the arrangement position of the focus lens 21 in the focused state in the control information already obtained in the illuminating light before the switch including a first spectral characteristic, by a predetermined value according to a difference between the first spectral characteristic and a second spectral characteristic included in the illuminating light after the switch. Therefore, according to the endoscope system 1 configured to execute such a process, complication of work regarding the focus adjustment can be reduced, and focus adjustment according to the spectral characteristic of the illuminating light can be performed.

By the way, a case can also be considered in which not only the rigid endoscope 10, but also, for example, a fiberscope formed by replacing the relay optical system inside of the cylindrical body portion 11 and the grasping portion 12 with a fiber bundle is connected to the camera unit 20 of the endoscope system 1. Therefore, according to the present embodiment, the endoscope system 1 may be configured so that, for example, the CPU 44 calculates the contrast value according to the detection result of the luminance value of step S3 of Fig. 4A, the CPU 44 identifies which one of the rigid endoscope 10 and the fiberscope will be used at the same time as the camera unit 20 based on the calculated contrast value, and the CPU 44 outputs a lens drive control signal according to the identified result to the lens drive section 22. Alternatively, according to the present embodiment, for example, the CPU 44 may identify which one of the rigid endoscope 10 and the fiberscope will be used at the same time as the camera unit 20, and the CPU 44 may output a lens drive control signal according to the identified result to the lens drive section 22 in the process of step S12 of Fig. 6A. Furthermore, according to the present embodiment, the endoscope system 1 may be configured to execute a process described below when an identification result indicating that the fiberscope will be used at the same time as the camera unit 20 is obtained.

On the other hand, when the fiberscope is used at the same time as the camera unit 20, the arrangement position of the focus lens 21 in which the optical image picked up by the image pickup device 23 is in the focused state is not deviated, because focus adjustment is performed for an end face of the fiberscope closer to the eyepiece section 14. Therefore, according to the present embodiment, for example, when an identification result indicating that the fiberscope will be used at the same time as the camera unit 20 is obtained, the processes of step S7 of Fig. 4B and step S15 of Fig. 6B may be executed only before lapse of a certain time from a predetermined timing. More specifically, for example, the processes of step S7 of Fig. 4B and step S15 of Fig. 6B may be executed only before lapse of a certain time from a timing in which the identification result indicating that the fiberscope will be used at the same time as the camera unit 20 is obtained. Then, according to the endoscope system 1 configured to perform the action, the lens drive control signal is (generated and) outputted only before lapse of a certain time from a predetermined timing when the identification result indicating that the fiberscope will be used at the same time as the camera unit 20 is obtained. Therefore, an increase in power consumption caused by output of the lens drive control signal can be suppressed.

Further, according to the present embodiment, for example, when an identification result indicating that the fiberscope will be used at the same time as the camera unit 20 is obtained, the CPU 44 may be configured to output a lens drive control signal for moving the focus lens 21 to an arrangement position shifted by a predetermined amount from the arrangement position in which the contrast value of the image data generated by the image pickup signal input section 41 is a maximum value. Then, according to the endoscope system 1 configured to perform the action, moire generated in an image obtained when the fiberscope is used to pick up an image of the observed object can be reduced.

Further, according to the present embodiment, for example, when an identification result indicating that the fiberscope will be used at the same time as the camera unit 20 is obtained, the image processing section 43 may execute image processing of applying a blur to a boundary line of white (bright section) and black (dark section) in the image data generated by the image pickup signal input section 41. Then, according to the endoscope system 1 configured to execute the image processing, moire generated in an image obtained when the fiberscope is used to pick up an image of the observed object can be reduced.

On the other hand, according to the present embodiment, for example, in a configuration in which a liquid crystal plate (not shown) that receives the light passing through the image pickup lens group of the camera unit 20 and a birefringent plate (not shown) that receives the light passing through the liquid crystal plate are arranged on a front surface of the image pickup device 23, a process described below or the like may be executed when the identification result indicating that the fiberscope will be used at the same time as the camera unit 20 is obtained.

Based on a frame rate of the image pickup device 23, the CPU 44 drives the liquid crystal plate so that a state in which a first optical image without pixel deviation relative to each pixel position of the image pickup device 23 is picked up and a state in which a second optical image with a pixel pitch shifted by 1/2 pixel in a horizontal direction and a vertical direction from each pixel position of the image pickup device 23 is picked up are switched during a period equivalent to one frame. Then, along with the action of the CPU 44, the image pickup signal input section 41 generates image data IG1 according to the first optical image described above and image data IG2 according to the second optical image described above during the period equivalent to one frame.

Subsequently, the image processing section 43 superimposes the image data IG1 and IG2 outputted from the image pickup signal input section 41 to generate image data IG3.

Furthermore, the image processing section 43 applies pixel interpolation processing to the generated image data IG3 as described above to generate image data IG4 with a pixel pitch 1/2 times the pixel pitch in the image data IG1 and IG2 and with the number of pixels four times the number of pixels in the image data IG1 and IG2.

Then, for example, the image processing section 43 generates image data IG5 by applying, to the image data IG4, a process of thinning out the pixels so that the number of pixels becomes the same as the number of pixels in the image data IG1 and IG2 and outputs the generated image data IG5 to the display control section 45. According to the action of the image processing section 43, an image corresponding to the image data IG5 is displayed on the monitor 5.

Here, calculation of the following equation (1) can be performed to obtain a moire period TMA equivalent to a pitch of moire components included in the image data IG1 and IG2, wherein P is the pixel pitch in the image data IG1 and IG2, and furthermore, P+$\delta$P (0<$\delta$P<P) is a pitch between respective fibers in the fiber bundle provided in the fiberscope.

$$TMA = P^2/\delta P \qquad ... (1)$$

Further, the pixel pitch P/2 of the image data IG4 can be assigned to the equation (1) to perform calculation of the following equation (2) to obtain a moire period TMB equivalent to a pitch of moire components included in the image data IG4.

$$TMB = (P/2)^2/\delta P = P^2/4\delta P \qquad ... (2)$$

Further, a relationship as indicated by the following equations (3) and (4) is satisfied, wherein FMA is a spatial frequency of the moire components included in the image data IG1 and IG2, and FMB is a spatial frequency of the moire components included in the image data IG4.

$$FMA = 1/TMA = \delta P/P^2 \qquad ... (3)$$

$$FMB = 1/TMB = 4\times(\delta P/P^2) = 4\times FMA \qquad ... (4)$$

That is, in the image data IG5, the moire components are moved to a highfrequency side compared to the image data IG1 and IG2. Therefore, according to the endoscope system 1 configured to generate the image data IG5, visibility of moire displayed on the monitor 5 can be reduced, while degradation of resolution is suppressed.

Note that according to the processes described above, for example, the spatial frequency FMB of the moire components may be removed by applying a low-pass filter to the image data IG4 before the image data IG5 is generated by thinning out the pixels of the image data IG4. Then, according to the endoscope system 1 configured to generate the image data IG5 in a state in which the spatial frequency FMB is removed, display of moire on the monitor 5 can be substantially prevented, while degradation of resolution is suppressed.

Note that the present invention is not limited to the embodiment described above, and various changes and applications are obviously possible without departing from the scope of the invention.

The present application is filed on the basis of claiming the benefit of priority from Japanese Patent Application No. 2013-123886, filed June 12, 2013 in Japan, and the disclosed contents are incorporated in the present specification, claims, and drawings by reference.

**Claims**

1. An endoscope system comprising:

   an image pickup apparatus in which an insertion portion comprising an objective optical system is detachable, the image pickup apparatus being configured to pick up an optical image of light from an observed object transmitted by the objective optical system of the insertion portion and to output the optical image as an image pickup signal;
   a focus changing section provided in the image pickup apparatus and comprising a drive section that can change focus of the image pickup apparatus;
   a control section configured to generate a control signal for automatically controlling the optical image in a focused state for the drive section of the focus changing section in a state in which the insertion portion is fitted to the image pickup apparatus; and
   an optical characteristic acquiring section configured to acquire an optical characteristic of the insertion portion used by the control section to generate the control signal.

2. The endoscope system according to claim 1, further comprising
   a frequency component specifying section that specifies a frequency component in which a contrast value of an image is equal to or greater than a predetermined threshold, the image being obtained when an image of a predetermined chart is picked up in the state in which the insertion portion is fitted to the image pickup apparatus, wherein the optical characteristic acquiring section acquires the optical characteristic of the insertion portion corresponding to the frequency component specified by the frequency component specifying section.

3. The endoscope system according to claim 1, further comprising:

   a storage section storing a database including a plurality of data associating a type of the insertion portion and control information including the optical characteristic of the insertion portion; and
   an identification section configured to identify the type of the insertion portion used at a same time as the image pickup apparatus based on one of an image generated according to the image pickup signal and information included in a wireless signal transmitted from a wireless transmission section provided in a predetermined member detachably fitted to the insertion portion, wherein
   the optical characteristic acquiring section acquires the control information corresponding to the type of the insertion portion identified by the identification section from the database to acquire the optical characteristic corresponding to the type of the insertion portion identified by the identification section.

4. The endoscope system according to claim 3, wherein
   when an image of a predetermined code displayed on an outer surface of the insertion portion is picked up, the identification section reads the predetermined code included in the picked up image to identify the type of the insertion portion.

5. The endoscope system according to claim 3, wherein
   the identification section calculates the contrast value of the image obtained when an image of a predetermined chart is picked up in the state in which the insertion portion is fitted to the image pickup apparatus and identifies the type of the insertion portion based on the calculated contrast value.

6. The endoscope system according to claim 3, wherein
   the control section sets an evaluation region corresponding to the type of the insertion portion identified by the identification section in the image generated according to the image pickup signal and generates the control signal for putting the set evaluation region into the focused state.

7. The endoscope system according to claim 3, wherein
   the control section executes a process for outputting, to the focus adjustment section, the control signal according to a spectral characteristic of illuminating light applied to the observed object.

8. The endoscope system according to claim 3, wherein
   the control section acts to output the control signal to the focus adjustment section only before lapse of a certain time from a predetermined timing, when the identification section obtains an identification result indicating that an optical transfer section that is provided in the insertion portion and that transfers light used by the image pickup apparatus to pick up the optical image of the observed object is formed by a fiber bundle.

9. The endoscope system according to claim 3, wherein
the control section generates the control signal for moving a focus lens included in the focus adjustment section to an arrangement position shifted by a predetermined amount from an arrangement position in which the contrast value of the image generated according to the image pickup signal is a maximum value, when the identification section obtains the identification result indicating that the optical transfer section that is provided in the insertion portion and that transfers the light used by the image pickup apparatus to pick up the optical image of the observed object is formed by a fiber bundle.

# FIG. 1

# FIG. 2

# FIG. 3

# FIG. 4A

START

APPLY POWER — S1

PRESS AF SWITCH — S2

DETECT LUMINANCE VALUE OF OBLIQUE EDGE PART
IN PLURALITY OF FREQUENCY COMPONENTS — S3

SPECIFY FREQUENCY COMPONENT FC1
IN WHICH CONTRAST VALUE
IS EQUAL TO OR GREATER THAN THRESHOLD TH1 — S4

ACQUIRE CONTROL INFORMATION
CORRESPONDING TO FREQUENCY COMPONENT FC1 — S5

END

# FIG. 4B

```
                    ┌─────────────┐
                    │    START    │
                    └──────┬──────┘
                           │
                           ▼
        ┌──────────────────────────────────────┐
        │  GENERATE LENS DRIVE CONTROL SIGNAL   │
        │        FOR SETTING FOCUSED STATE      │────S6
        │       IN FREQUENCY COMPONENT FC1      │
        └──────────────────┬───────────────────┘
                           │
                           ▼
                                                    S7
        ╱─────────────────────────────────────╲
        ╲   CONTRAST VALUE SMALLER             ╱
   YES  ╱    THAN THRESHOLD THL?               ╲
        ╲─────────────────────────────────────╱
                           │
                          NO
```

# FIG. 5

# FIG. 6A

```
        ┌─────────────┐
        │    START    │
        └─────────────┘
               │
               ▼
┌─────────────────────────────────────┐
│             APPLY POWER             │──S11
└─────────────────────────────────────┘
               │
               ▼
┌─────────────────────────────────────┐
│     IDENTIFY TYPE OF RIGID ENDOSCOPE │──S12
└─────────────────────────────────────┘
               │
               ▼
┌─────────────────────────────────────┐
│ ACQUIRE CONTROL INFORMATION CORRESPONDING │──S13
│       TO TYPE OF RIGID ENDOSCOPE        │
└─────────────────────────────────────┘
               │
               ▼
        ┌─────────────┐
        │     END     │
        └─────────────┘
```

# FIG. 6B

START

GENERATE LENS DRIVE CONTROL SIGNAL
FOR SETTING FOCUSED STATE ACCORDING
TO TYPE OF RIGID ENDOSCOPE — S14

CONTRAST VALUE SMALLER
THAN THRESHOLD THM? — S15

YES

NO

# FIG. 7

# FIG. 8

# FIG. 9

15

61

# FIG. 10

12    15    61    14

13

## INTERNATIONAL SEARCH REPORT

| | International application No. |
| --- | --- |
| | PCT/JP2014/065333 |

**A. CLASSIFICATION OF SUBJECT MATTER**
*A61B1/04*(2006.01)i, *A61B1/00*(2006.01)i, *G02B23/24*(2006.01)i

According to International Patent Classification (IPC) or to both national classification and IPC

**B. FIELDS SEARCHED**

Minimum documentation searched (classification system followed by classification symbols)
A61B1/00-1/32, G02B23/24-23/26

Documentation searched other than minimum documentation to the extent that such documents are included in the fields searched
Jitsuyo Shinan Koho        1922-1996   Jitsuyo Shinan Toroku Koho    1996-2014
Kokai Jitsuyo Shinan Koho   1971-2014   Toroku Jitsuyo Shinan Koho    1994-2014

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | JP 2-207213 A (Olympus Optical Co., Ltd.),<br>16 August 1990 (16.08.1990),<br>page 4, lower right column, line 9 to page 6,<br>upper right column, line 10; fig. 8<br>& US 4905668 A | 1,3<br>2,4-9 |

☐ Further documents are listed in the continuation of Box C.   ☐ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>    03 September, 2014 (03.09.14) | Date of mailing of the international search report<br>    16 September, 2014 (16.09.14) |
| --- | --- |
| Name and mailing address of the ISA/<br>    Japanese Patent Office | Authorized officer |
| Facsimile No. | Telephone No. |

Form PCT/ISA/210 (second sheet) (July 2009)

**EP 2 957 217 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005334462 A
- JP 2011147707 A
- JP 2013123886 A